# EUROPEAN PATENT APPLICATION

(11) **EP 4 520 182 A1**
(43) Date of publication of application: **12.03.2025**
(21) Application number: 24198607.4
(22) Date of filing: 05.09.2024
(51) Int. Cl.: A23J 1/00, A23J 1/12, A23J 3/14, A23J 3/18, A23K 10/38

(54) **METHOD AND SYSTEM FOR INCREASING THE YIELD AND/OR PURITY OF PROTEIN AND OIL FROM A GRAIN PROTEIN AND GRAIN OIL RECOVERY SYSTEM**

(30) Priority: 07.09.2023 US 202363581048 P
(71) Applicant: Fluid Quip Technologies, LLC, Cedar Rapids, Iowa 52402 (US)
(72) Inventor: Jakel, Neal, Cedar Rapids, IA, 52403 (US); Kwik, John, Bellbrook, OH, 45305 (US); Kempf, Chris, Cedar Rapids, IA, 52402 (US); Runge, Brian, Cedar Rapids, IA, 52402 (US)
(74) Representative: Wallinger Ricker Schlotter Tostmann

(57) **Abstract**

An improved dry grind method and system of alcohol and/or biochemical production for increasing the yield and/or purity of protein and/or oil in facilities that produce alcohol (e.g., ethanol) from corn or other cereal grains. In one embodiment, the method includes membrane filtration of a liquid portion from a high protein slurry that has been separated into the liquid portion and a high protein wet cake portion initially derived from a whole stillage byproduct. The liquid portion may be cooled in a heat exchanger to a temperature suitable for a desired membrane filter. Solids, dissolved proteins, and oils are separated by the membrane and concentrated in a retentate stream. Low molecular weight dissolved solids pass through the membrane as permeate. The proteins and oil from the retentate are returned to prior separation equipment that produced the high protein slurry and will be separated by said equipment increasing protein and/or oil yields and/or purity.

## Description

### Technical Field

The present invention relates to dry grind methods and systems of alcohol production and increasing the yield and/or purity of both protein and oil from systems that separate protein and oil from a whole stillage byproduct in a plant that derives a biochemical, such as alcohol (e.g., ethanol), from grain.

### Background

Dry milling ethanol plants generally convert corn and/or other grains into only three products, i.e., ethanol, distillers grain oil, and distiller's grains with solubles. A typical grain dry milling process consists of four major steps: grain handling and milling, liquefaction and saccharification, fermentation and distillation, and co-product recovery. Grain handling and milling is the step in which the grain is brought into the plant and ground to promote better conversion of starch to glucose. Liquefaction is the step of converting solids, such as starch, to a flowable liquid producing oligosaccharides and saccharification is where the oligosaccharides are converted into single glucose molecules. Fermentation is the process of yeast or bacteria, or as clostridia, for example, converting glucose into a biofuel or a biochemical/biomolecule, such as ethanol. Distillation is the process of removing the biofuel or biochemical/biomolecule, such as ethanol, from the fermentation product. Co-product recovery is the step in which the grain byproducts are de-watered and made ready for market. There are many known chemical and biological conversion processes known in the art that utilize yeast, bacteria, or the like to convert glucose/sugar to other biofuels and biochemical/biomolecule components like ethanol, for example.

The recovery of alcohol, e.g., butanol, ethanol (a natural co-product), etc., and other similar compounds, generally begins with the beer (spent fermentation broth) being sent to a distillation system. With distillation, ethanol is typically separated from the rest of the beer through a set of stepwise vaporizations and condensations. The beer less the alcohol extracted through distillation is known as whole stillage, which contains a slurry of the spent grains including grain protein, fiber, oil, minerals, sugars, and fermentation agent. This byproduct is too diluted to be of much value at this point and is further processed to provide the distiller's grains with solubles.

In typical processing, when the whole stillage leaves the distillation column, it is generally subjected at the back end of the process to a decanter centrifuge to separate insoluble solids or "wet cake", which includes mostly fiber, from the liquid or "thin stillage", which includes, e.g., protein, fine fiber, oil, and amino acids. After separation, the thin stillage moves to evaporators to boil away moisture, leaving a thick syrup that contains the soluble (dissolved) solids. The concentrated syrup is typically mixed with the wet cake, and the mixture may be sold to beef and dairy feedlots as distillers wet grain with solubles (DWGS). Alternatively, the wet cake and concentrated syrup mixture may be dried in a drying process and sold as distillers dried grain with solubles (DDGS). The resulting DDGS generally has a crude protein content of about 29% and is a useful feed for cattle and other ruminants due to its protein and fiber content.

While DDGS and DWGS provide a critical secondary revenue stream that offsets a portion of the overall ethanol production cost, it would be beneficial to provide a method and system where a high protein grain product, e.g., a high protein grain product, can be obtained from the whole stillage to be sold at a higher cost per ton than DDGS or DWGS and/or to further enhance oil recovery, which can be sold as a separate high value product.

### Summary of the Invention

The present invention is directed to a method and system for increasing the yield and/or purity of protein and/or oil from a system that produces protein and oil from dry milling of grains including, for example, corn and wheat.

In one embodiment, a method for increasing the yield and/or purity of protein and oil from a whole stillage byproduct from a biochemical process includes membrane filtration of a liquid portion decanted from a high protein slurry that has been separated into the liquid portion and a high protein wet cake portion. The liquid portion may be optionally cooled in a heat exchanger to a temperature suitable for the selected/desired membrane filter. The optionally cooled liquid portion/stream is pumped into/sent to a membrane filter. Solids, high molecular weight dissolved proteins, and oils are rejected/separated by the membrane filter and concentrated in a retentate stream. Low molecular weight dissolved solids will pass through the membrane as the permeate. The concentrated proteins and oil from the retentate will be returned to prior separation equipment (e.g., a centrifuge) that produced the high protein slurry in the method and will be captured/separated by said equipment increasing yields and/or purity.

In another embodiment, a method for increasing protein yield and/or purity from a whole stillage byproduct produced in a biofuel and/or biochemical production process is provided that includes separating a whole stillage byproduct into a fiber portion and a liquid slurry, which includes initial free oil and protein particles. The method further includes adding at least a portion of a retentate, which includes residual protein particles and residual free oil, that is received from a later step in the method to the liquid slurry, and separating the liquid slurry into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion. Thereafter, a wash water is separately added to the separated protein portion followed by dewatering the protein portion to provide a liquid fraction and a protein wet cake fraction, including the combined protein particles. And then, the liquid fraction is filtered to provide a retentate, including residual protein particles and residual free oil, and a permeate, wherein at least a portion of the retentate defines the portion of the retentate received from the later step in the method, and wherein the protein wet cake fraction defines a high protein meal that includes at least 48 wt % protein on a dry basis.

In another embodiment, a method for increasing protein yield and/or purity from a whole stillage byproduct produced in a biofuel and/or biochemical production process is provided that includes separating a whole stillage byproduct into a fiber portion and a liquid slurry, which includes initial free oil and protein particles. The method further includes adding at least a portion of a retentate, which includes residual protein particles and residual free oil, that is received from a later step in the method to the liquid slurry, and separating the liquid slurry, via weights, into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion, thereafter, a wash water is separately added to the separated protein portion followed by dewatering the protein portion to provide a liquid fraction and a protein wet cake fraction, including the combined protein particles. And then, the liquid fraction is cooled, via a heat exchanger, followed by filtering the cooled liquid fraction, via a membrane, to provide a retentate, including residual protein particles and residual free oil, and a permeate, wherein at least a portion of the retentate defines the portion of the retentate received from the later step in the method, and wherein the protein wet cake fraction defines a high protein meal that includes from 48 wt % to 70 wt % protein on a dry basis.

In yet another embodiment, a system for increasing protein yield and/or purity from a whole stillage byproduct produced in a biofuel and/or biochemical production process is provided that includes a first apparatus that receives a whole stillage byproduct produced in a biofuel and/or biochemical production process, wherein the first apparatus separates the whole stillage byproduct into a fiber portion and a liquid slurry, which includes initial free oil and protein particles. A second apparatus is situated after the first apparatus, the second apparatus receives the liquid slurry from the first apparatus and whereat at least a portion of a retentate, which includes residual protein particles and residual free oil, is added to the liquid slurry from a later apparatus in the system. The second apparatus is configured to separate the liquid slurry into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion. A third apparatus is situated after the second apparatus and that receives the protein portion from the second apparatus and whereat wash water is added to the protein portion. The third apparatus is configured to dewater the protein portion to provide a liquid fraction and a protein wet cake fraction, including the combined protein particles. A filtration device is situated after the third apparatus and receives the liquid fraction. The filter device is configured to separate the liquid fraction into a retentate, including residual protein particles and residual free oil, and a permeate, wherein at least a portion of the retentate defines the portion of the retentate received at the second apparatus from the later apparatus in the system and wherein the protein wet cake fraction defines a high protein meal that includes at least 48 wt % protein on a dry basis.

### Brief Description of the Drawings

The accompanying drawings, which are incorporated in and constitute a part of this specification illustrate embodiments of the invention and, together with the general description of the invention given above and the detailed description given below, serve to explain the principles of the invention. Similar reference numerals are used to indicate similar features throughout the various figures of the drawings.
FIG. 1 is a flow diagram of a typical dry grind alcohol production process;
FIG. 2 is a flow diagram of a prior art method and system for producing a high protein meal from a whole stillage byproduct produced via a dry milling process of grain for making ethanol; and
FIG. 3 is a flow diagram of a method and system for increasing the yield and/or purity of protein and/or oil by membrane filtration in a dry grind biochemical process in accordance with an embodiment of the invention.

### Detailed Description of Specific Embodiments

The present invention relates to dry grind methods and systems of alcohol and/or biochemical production for increasing the yield and/or purity of protein and/or oil from systems that separate protein and oil from whole stillage in a plant that derives alcohol (e.g., ethanol) from corn, wheat, or other cereal grains.

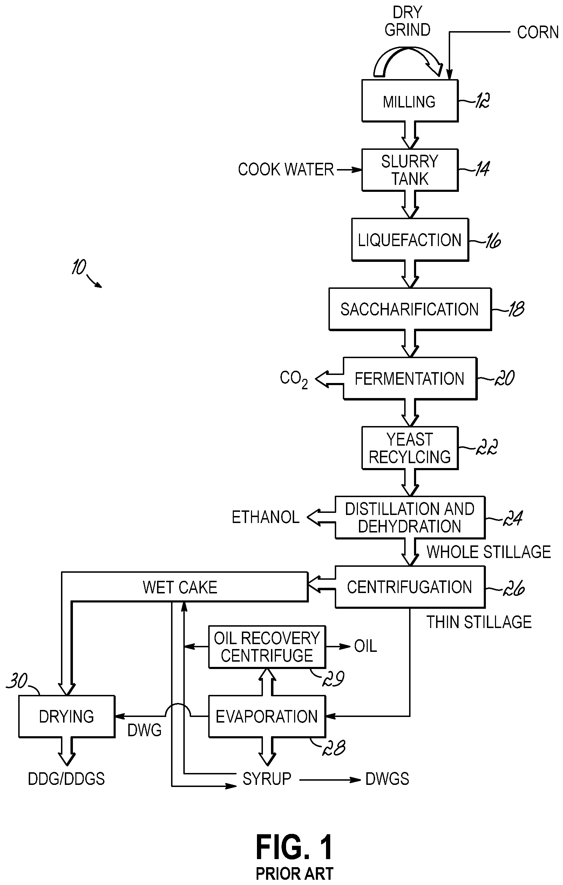

Fig. 1 shows a flow diagram of a typical dry grind alcohol (e.g., ethanol) production method 10. Although virtually any type and quality of grain, such as but not limited to sorghum, wheat, triticale, barley, rye, tapioca, cassava, potato, pea and other starch and/or oil containing grains and/or legumes can be used to produce ethanol and/or a biochemical/biomolecule, for example, the feedstock for this process is typically corn referred to as "No. 2 Yellow Dent Corn." Also, as a general reference point, the dry grind method 10 can be divided into a front end and a back end. The part of the method 10 that occurs prior to distillation 24 is considered the "front end," and the part of the method 10 that occurs after distillation 24 is considered the "back end." To that end, the front end of the dry grind method 10 begins with a milling step 12 in which dried whole corn kernels can be passed through hammer mills for grinding/milling into meal or a fine powder. The screen openings in the hammer mills or similar devices typically are of a size 6/64 to 9/64 inch, or about 2.38 mm to 3.57 mm, but some plants can operate at less than or greater than these screen sizes. The resulting particle distribution yields a very widely spread, bell type curve, which includes particle sizes as small as 45 microns and as large as 2 mm to 3 mm. The majority of the particles tend to be in the range of 500 to 1200 microns, which is the "peak" of the bell curve. Other screen openings larger and smaller can be deployed in the hammer mills. Other milling devices such as roller mills or pin mills can also be utilized in the dry grain grinding step.

After the milling step 12, the ground meal is mixed with cook water to create a slurry at the slurry tank 14 and a commercial enzyme called alpha-amylase is typically added (not shown). Creating the slurry at the slurry tank 14 is followed by a liquefaction step 16 whereat the pH is adjusted to about 4.8 to 5.8 and the temperature maintained between about 50°C to 105°C so as to convert the insoluble starch in the slurry to soluble starch. The stream after the liquefaction step 16 has about 30% dry solids (DS) content, but can range from about 29-36%, with all the components contained in the corn kernels, including starch/sugars, protein, fiber, starch, germ, grit, oil, and salts, for example. Higher solids are achievable, but this requires extensive alpha amylase enzyme to rapidly breakdown the viscosity in the initial liquefaction step. There generally are several types of solids in the liquefaction stream: fiber, germ, and grit.

Liquefaction 16 may be followed by separate saccharification and fermentation steps, 18 and 20, respectively, although in most commercial dry grind ethanol processes, saccharification and fermentation can occur simultaneously. This single step is referred to in the industry as "Simultaneous Saccharification and Fermentation" (SSF). Both saccharification and SSF can take as long as about 50 to 60 hours. Gluco-Amylase enzyme is typically added to the fermentation step that facilitates the further breakdown of the starches and larger polysaccharides in to single monomer sugar molecules that the yeast consumes to produce ethanol (or other similar alcohols) and carbon dioxide. Yeast can optionally be recycled in a yeast recycling step 22 either during the fermentation process or at the very end of the fermentation process. Yeast produced during the fermentation process will pass through to the distillation and dehydration step 24. In addition to the gluco-amylase being added, other enzymes can be added (such as but not limited to phytase, protease, cellulase, hemicellulose, xylanase, beta-glucanase, and the like) that can further enhance the protein and oil recovery downstream. Subsequent to the fermentation step 20 is the distillation (and dehydration) step 24, which utilizes a still to recover the alcohol (e.g., ethanol).

Finally, a centrifugation step 26 involves centrifuging the residuals, i.e., "whole stillage", which includes the non-fermentable grain components (protein, oil, fiber, ash, and minerals, for example) and yeast yielded from the distillation and dehydration step 24 in order to separate the insoluble solids ("wet cake") from the liquid ("thin stillage"). The liquid from the centrifuge contains about 5% to 12% DS. The "wet cake" includes fiber, of which there generally are three types: (1) pericarp, with average particle sizes typically about 1 mm to 3 mm; (2) tipcap, with average particle sizes about 500 micron; (3) and fine fiber, with average particle sizes of about 250 microns. There may also be proteins and yeast bodies with a particle size of about 45 microns to about 300 microns. The fiber and other fractions may contain bound protein that is chemically and or physically attached to the fiber and other fraction.

The thin stillage typically enters evaporators in an evaporation step 28 in order to boil or flash away moisture, leaving a thick syrup which contains the soluble (dissolved) solids (mainly protein and starches/sugars) from the fermentation (25 to 40% dry solids) along with residual oil and fine fiber. The concentrated slurry can be sent to a centrifuge to separate the oil from the syrup in an oil recovery step 29. The oil can be sold as a separate high value product. The oil yield is normally about 0.7 lb/bu of corn with elevated free fatty acids content compared to traditional wet mill corn oil. This oil yield recovers only about 1/3 of the oil in the corn, with part of the oil passing with the syrup stream and the remainder being lost with the fiber/wet cake stream. About one-half of the oil inside the corn kernel remains inside the germ after the distillation and dehydration step 24, which cannot be separated in the typical dry grind process using centrifuges as the oil is bound, not free. The free fatty acids content, which is created when the oil is heated and exposed to oxygen throughout the front and back-end process, reduces the value of the oil. The (de-oil) centrifuge only removes less than 50% because the protein and oil make an emulsion, which cannot be satisfactorily separated without the use of chemicals or added mechanical separation unit operations.

The syrup, which has more than 10% oil, can be mixed with the centrifuged wet cake, and the mixture may be sold to beef and dairy feedlots as Distillers Wet Grain with Solubles (DWGS). Alternatively, the wet cake and concentrated syrup mixture may be dried in a drying step 30 and sold as Distillers Dried Grain with Solubles (DDGS) to dairy and beef feedlots. This DDGS has all the corn and yeast protein and about 67% of the oil in the starting corn material. But the value of DDGS is low due to the high percentage of fiber, and in some cases the oil is a hindrance to animal digestion and lactating cow milk quality.

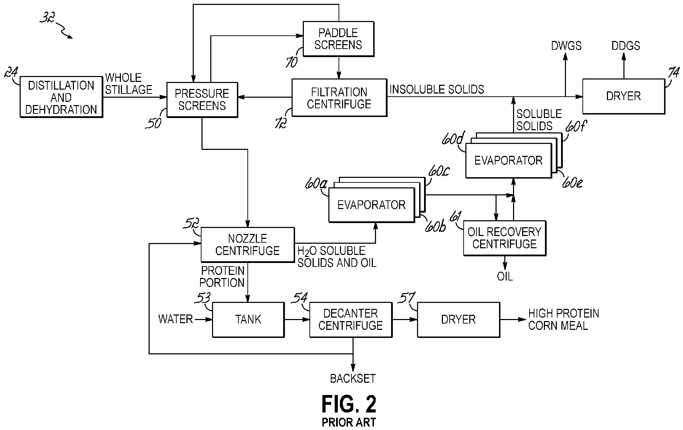

Fig. 2 shows a prior art method and system for producing a high protein corn meal, collectively numeral 32, from a whole stillage byproduct such as produced in a typical corn dry-milling method and system 10 like that just described in Fig. 1. The method begins with whole stillage from the distillation and dehydration step 24 of the dry grind ethanol method. The whole stillage is passed over one or more pressure screens 50 with openings sized to permit the passage of protein particles and yeast cells but reject/prevent the passage of most fiber particles.

The material that passes through the pressure screens in step 50 is higher in protein due, for example, to the exclusion of fiber by the pressure screens. This protein rich slurry is sent to and further separated by one or more disc nozzle centrifuges in step 52, which can be arranged in series or parallel. In these centrifuges, the denser material including the protein particles can be concentrated in a high density underflow stream, which is referred to as a protein portion. The lighter material including most of the oil and some of the lighter (fine) fiber material passes to a lower density overflow stream. This overflow stream, which includes oil and water-soluble solids, is sent to a set of three evaporators 60a, 60b, and 60c, as are known in the art, whereat water is removed to begin to thicken the water soluble solids and oil stream to a high solids syrup.

Thereafter, the overflow stream can be piped and subjected to an optional oil recovery centrifuge 67 so that oil can be removed therefrom. In one example, the final recovered oil product can include between about 40 wt% to about 60 wt% of the total corn oil in the corn. The remainder of the overflow stream can be piped and subjected to another set of three evaporators 60d, 60e, and 60f whereat water is further evaporated to yield the high solids syrup. While Fig. 2 shows the overflow stream being subjected to two sets of three evaporators, 60a-c and 60d-f, it should be understood that the number of evaporators and sets thereof can be varied, i.e., can be more or less, from that shown depending on the particular application and result desired.

The underflow stream from the disc nozzle centrifuge 52, which defines the protein portion, is collected in a tank 53. Water can be added to the tank to dilute the underflow stream and help in washing soluble non protein solids from the high protein portion/slurry increasing the purity of a final protein meal. The underflow stream can be separated into a liquid fraction and a protein wet cake fraction at one or more decanting centrifuges 54 (e.g., a decanter centrifuge). The protein wet cake contains most of the protein and can be dried in a dryer 57 to make a high protein meal product. The liquid product or centrate is returned to the front end of the alcohol (e.g., ethanol) process as backset and used to slurry corn flour such as at slurry tank 14. In addition, at least a portion of the centrate may be returned back to the nozzle centrifuge 52.

Following the fiber stream exiting the pressure screen at 50, the high fiber wet cake that is rejected/separated by the pressure screen 50 can be (re)slurried and passed through one or more paddle screens 70. The wet cake that exits the paddle screens 70 can again be (re)slurried and then run through one or more filtration centrifuges 72. The centrate from both the paddle screens 70 and the filtration centrifuges 72 carries as yet unrecovered protein particles. These centrate streams are returned to the pressure screen 50 so that protein and oil that may have been missed on the first pass over the screens can be recovered by passing through the pressure screen 50 and on to the nozzle centrifuge 52.

The wet fiber cake that is separated from the liquid stream at the filtration centrifuge 72 is mixed with syrup from the evaporators 60f, 60e, 60d and is either sold as Distillers Wet Grains with Solubles (DWGS), or is run through a dryer 74 and is sold as Distillers Dried Grains with Solubles (DDGS).

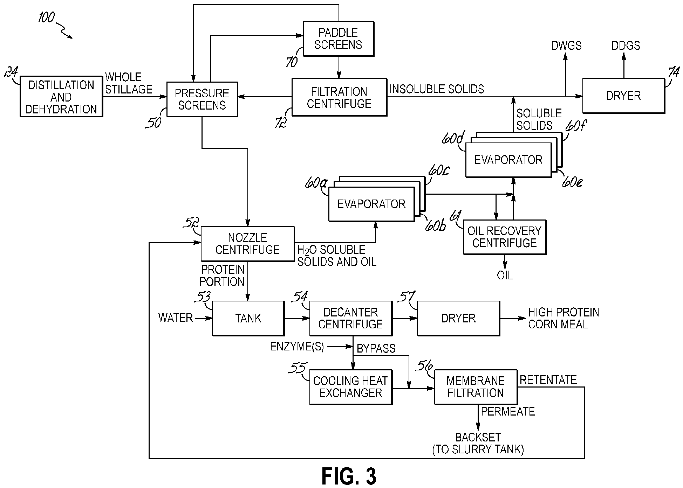

In accordance with the present invention, Fig. 3 shows one embodiment of a method and system for increasing the yield and/or purity of protein and oil in a dry grind process, collectively numeral 100, like the dry grind process 32 just described in Fig. 2. Certain reference numerals used in Fig. 2 are used here to represent like devices and/or steps in the method and system 100. The variations of the embodiment of Fig. 3 are discussed hereinbelow.

As shown in Fig, 3, in this embodiment, the liquid fraction from the decanter centrifuge 54 can be optionally cooled in a heat exchanger 55, by means and methods known in the art, to a temperature suitable for operation with a selected/desired type of membrane filtration device 56 with the liquid fraction (cooled or not) ultimately being subjected to said membrane filtration device 56, as is further discussed below. If the optional cooling heat exchanger 55 is not required, it may be eliminated altogether or the liquid fraction may be bypassed, manually or automatically by means and methods known in the art, around the cooling heat exchanger 55. In one example, one or more enzymes and/or other chemicals can be added to the liquid fraction before or after the optional cooling heat exchanger 55, such as but not limited to amylase, alpha-amylase, glucoamylase, fungal, phytase, protease (e.g., serine protease), cellobiose, cellulase, hemicellulase, xylanase, gluconase, transglutaminase (e.g. microbial), and the like to further enhance protein and/or oil recovery. For example, the enzyme(s) may aid in binding protein particles together and/or free oil together, which can result in a greater concentration of protein and/or oil in the retentate after filtering of the liquid fraction via the membrane filtration device 56 and, thus, ultimately provide a greater overall yield and/or purity of high protein grain meal and/or oil.

With respect to the membrane filtration device 56, a wide variety of filter media as the membrane filtration devices 56 are suitable for use. In one example, any membrane filter with a pore size suitably small enough to exclude the passing of solids (including, for example, protein solids) and/or higher molecular weight dissolved solids can be used. Such membrane filters can include polymer membranes, sintered metal (e.g., stainless steel) filters, coated (e.g., polymeric coated) filters, or ceramic filters, and the like. In one example, the pore size required for this type of application falls into the area normally described as microfiltration. In another example, ultrafiltration, which is the next step smaller in pore size, could also be used if the microfiltration method does not desirably reject/separate enough of the protein solids and/or oil material that needs to be recycled for recovery. In other words, the membrane filtration device 56 can be a microfilter, an ultrafilter, or a nanofilter. In one example, there may be more than one membrane filtration device 56, which may be the same or different type of device and can be arranged in series or parallel. In one embodiment, the membrane filtration device 56 includes a microfiltration device followed by an ultrafiltration device. In still another example, the membrane filtration device 56 can include a reverse osmosis (RO) filter, which may be used in combination with other RO filters and/or other membrane filtration devices 56. The pore size required for this type of application can fall into the nanofiltration size and smaller, e.g., 0.001 to 0.0001 microns. With the use of RO filtration, the permeate may be clean enough for use as wash water earlier in the method and system 100, if so desired.

In one example, the filtration membrane selected could be a flat sheet membrane. In another example, the membrane for the membrane filtration device 56 can be a spiral wound membrane since that design is more compact for a given filter surface area. Metal and ceramic filter elements also have lower surface area per volume and can require a larger footprint to install.

For polymer based filter membranes, the temperature of the liquid fraction being filtered may need to be cooled from a normal operating temperature of approximately 190°F to a filtration temp of as low as 120°F. In another example, the temperature of the liquid fraction being filtered may need to be cooled down to about 100°F. The optional cooling heat exchanger to be used in cooling the liquid fraction could be a shell and tube or a plate and frame type heat exchanger, as are known in the art. In one example, cooling tower water may be the most readily available media for the cold side of the optional cooling heat exchanger 55. Other process streams/stream liquid may be utilized along with or instead of cooling tower water for cooling the liquid fraction, as needed/desired.

If sintered metal, coated, or ceramic membrane filters are selected, the optional cooling heat exchanger may be bypassed or eliminated altogether. These types of filters can be capable of handling a hot 190°F feed material and, thus, the liquid fraction/centrate can be pumped directly to the membrane filtration device 56.

The membrane filtration device 56 could be set up for either cross flow or perpendicular flow. A cross flow installation typically will have a longer run time between filter change outs due to the scouring nature of flow across the filter surface.

Solids, high molecular weight dissolved solids, including proteins and oils are rejected/separated by the filter media and are concentrated in the retentate. Low molecular weight dissolved solids will pass through the filter/membrane filtration device 56 (along with water) as the permeate, which can be returned to the front end as backset and used to slurry the ground corn such as at slurry tank 14. The concentrated proteins and oil from the retentate will be returned to the nozzle centrifuge 52. Additional sources or uses of the permeate can be deployed within the dry-grind process or utilized within other processes unrelated to the base dry-grind process.

At the nozzle centrifuge 52, the protein will be separated and go with the dense phase underflow of the nozzle centrifuge 52 and the oil will go with the light phase overflow of the nozzle centrifuge 52 and on to the evaporators 60a-e, as discussed above, such as for oil recovery via the oil recovery centrifuge 61. This additional material, i.e., protein and oil, being captured and recycled/returned back to the nozzle centrifuge 52 will further desirably increase the yield and/or purity of protein in the resulting high protein meal product and oil by way of the oil recovery centrifuge 61. In one example, the final recovered oil product between the evaporators 60a-f can include from about 40 wt% to about 80 wt% of the total corn oil in the corn. In another example, the final recovered oil product can include from about 50 wt% to about 80 wt%. In still another example, the final recovered oil product can include from about 60 wt% to about 80 wt%. In still another example, the final recovered oil product can include from about 70 wt% to about 80 wt%. In another example, the final recovered oil product can include from about 40 wt% to about 60 wt% or from about 50 wt% to about 70 wt%. And the resulting protein product that defines high protein grain (e.g., corn) meal can include at least 50 wt% protein on a dry basis and which may be sold as pig or chicken feed, for example. In another embodiment, the high protein meal includes at least 60 wt% protein on a dry basis. In still another embodiment, the high protein meal includes at least 70 wt% protein on a dry basis. In one embodiment, the high protein meal can include from about 48 wt% to about 70 wt% protein on a dry basis. In another embodiment, the high protein meal can include from about 50 wt% to about 70 wt% or from about 55 wt% to about 70 wt%. In still another embodiment, the high protein meal can include from about 60 wt% to about 70 wt%. In yet another embodiment, the high protein meal has about 70 wt% protein on a dry basis. In still another embodiment, the high protein meal can include from about 48 wt% to about 52 wt% or from about 50 wt% to about 60 wt%.

## Claims

1. A method for increasing protein yield and/or purity from a whole stillage byproduct produced in a biofuel and/or biochemical production process comprising:
separating a whole stillage byproduct, preferably via filtration, into a fiber portion and a liquid slurry, which includes initial free oil and protein particles;
adding at least a portion of a retentate, which includes residual protein particles and residual free oil, that is received from a later step in the method to the liquid slurry, and separating the liquid slurry into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion;
thereafter, separately adding a wash water to the separated protein portion followed by dewatering the protein portion to provide a liquid fraction and a protein wet cake fraction, including the combined protein particles; and
filtering the liquid fraction to provide a retentate, including residual protein particles and residual free oil, and a permeate, wherein at least a portion of the retentate defines the portion of the retentate received from the later step in the method, and
wherein the protein wet cake fraction defines a high protein meal that includes at least 48 wt % protein on a dry basis.

2. The method of claim 1, further comprising cooling the liquid fraction, preferably via a heat exchanger, further preferably to a temperature less than 87.8°C (190°F), prior to filtering the liquid fraction to provide the retentate and permeate.

3. The method of claim 1 wherein filtering the liquid fraction to provide a retentate and a permeate includes filtering via a membrane.

4. The method of claim 3, wherein the membrane is a flat sheet membrane or a spiral wound membrane; or wherein the membrane comprises a stainless steel sintered filter; or wherein the membrane comprises a coated polymeric filter; or wherein the membrane comprises a reverse osmosis filter; or wherein filtering of the liquid fraction is performed multiple times via different membranes arranged in series or parallel.

5. The method of claim 1, wherein filtering the liquid fraction to provide a retentate and a permeate includes microfiltering, ultrafiltering, or nanofiltering the liquid fraction; or wherein separating the liquid slurry includes separating the liquid slurry, via weights, into a protein portion, including combined initial and residual protein particles, and a water soluble solids portion, including combined initial and residual free oil.

6. The method of claim 1, wherein the addition of the retentate facilitates separation of a larger concentration of protein particles in the protein portion and free oil in the water soluble solids portion; and further comprising subjecting the separated water soluble solids portion to evaporation via an evaporator followed by separating the combined oil from the water soluble solids portion via an oil recovery centrifuge to provide an oil portion.

7. The method of claim 6, wherein the recovered oil portion includes from about 40 wt% to about 80 wt% of the total grain oil of the grain, preferably corn, used in the method.

8. The method of claim 1, further comprising utilizing the permeate as backset in a step earlier in the method; or drying the protein wet cake fraction; or adding one or more enzymes and/or other chemicals to the liquid fraction prior to filtering the liquid fraction.

9. The method of claim 1 wherein the biofuel and/or biochemical production process is an alcohol production process; or wherein the high protein meal includes from 48 wt % to 70 wt % protein on a dry basis.

10. A method for increasing protein yield and/or purity from a whole stillage byproduct produced in a biofuel and/or biochemical production process comprising:
separating a whole stillage byproduct into a fiber portion and a liquid slurry, which includes initial free oil and protein particles;
adding at least a portion of a retentate, which includes residual protein particles and residual free oil, that is received from a later step in the method to the liquid slurry, and separating the liquid slurry, via weights, into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion;
thereafter, separately adding a wash water to the separated protein portion followed by dewatering the protein portion to provide a liquid fraction and a protein wet cake fraction, including the combined protein particles; and
cooling the liquid fraction, via a heat exchanger, followed by filtering the cooled liquid fraction, via a membrane, to provide a retentate, including residual protein particles and residual free oil, and a permeate, wherein at least a portion of the retentate defines the portion of the retentate received from the later step in the method, and
wherein the protein wet cake fraction defines a high protein meal that includes from 48 wt % to 70 wt % protein on a dry basis.

11. A system for increasing protein yield and/or purity from a whole stillage byproduct produced in a biofuel and/or biochemical production process comprising:
a first apparatus that receives a whole stillage byproduct produced in a biofuel and/or biochemical production process, wherein the first apparatus separates the whole stillage byproduct into a fiber portion and a liquid slurry, which includes initial free oil and protein particles;
a second apparatus that is situated after the first apparatus, the second apparatus receives the liquid slurry from the first apparatus and whereat at least a portion of a retentate, which includes residual protein particles and residual free oil, is added to the liquid slurry from a later apparatus in the system, the second apparatus configured to separate the liquid slurry into a protein portion, including combined initial and residual protein particles, and a water soluble solids portion, including combined initial and residual free oil, wherein the addition of the retentate increases the overall concentration of protein particles and free oil in the liquid slurry and facilitates separation of a larger concentration of protein particles in the protein portion; and
a third apparatus that is situated after the second apparatus and that receives the protein portion from the second apparatus and whereat wash water is added to the protein portion, the third apparatus configured to dewater the protein portion to provide a liquid fraction and a protein wet cake fraction, including the combined protein particles; and
a filtration device that is situated after the third apparatus and that receives the liquid fraction, the filter device configured to separate the liquid fraction into a retentate, including residual protein particles and residual free oil, and a permeate, wherein at least a portion of the retentate defines the portion of the retentate received at the second apparatus from the later apparatus in the system, and
wherein the protein wet cake fraction defines a high protein meal that includes at least 48 wt % protein on a dry basis.

12. The system of claim 11, wherein the first apparatus is a pressure screen, the second apparatus is a nozzle centrifuge or a cyclone apparatus, the third apparatus is a centrifuge, and the filtration device is a membrane filtration device.

13. The system of claim 11, further comprising a cooling device, preferably a heat exchanger, that is situated after the third apparatus and before the filtration device, the cooling device configured to receive and cool the liquid fraction, preferably to a temperature less than 87.8°C (190°F), prior to being subjected to the filtration device.

14. The system of claim 11, wherein the filtration device is one of a microfiltration device, ultrafiltration device, or a nanofiltration device; or wherein the filtration device includes a reverse osmosis filter; or wherein the filtration device includes multiple filtration devices in a series or parallel arrangement and each configured to separate the liquid fraction into the retentate and permeate.

15. The system of claim 11, wherein the addition of the retentate facilitates separation of a larger concentration of protein particles in the protein portion and free oil in the water soluble solids portion; and further comprising an evaporator that is situated after the second apparatus and that is configured to receive and subject the separated water soluble solids portion to evaporation, and an oil recovery centrifuge situated after the evaporator and that is configured to receive and separate the combined oil from the water soluble solids portion to provide an oil portion; or further comprising a dryer situated after the third apparatus and configured to receive and dry the protein wet cake fraction.
